(19) Europäisches Patentamt
European Patent Office
Office européen des brevets

(11) Publication number: **0 571 700 A1**

(12) **EUROPEAN PATENT APPLICATION**

(21) Application number: **92850235.0**

(51) Int. Cl.[5]: **A61K 9/70**, A61L 15/00

(22) Date of filing: **07.10.92**

Amended claims in accordance with Rule 86 (2) EPC.

(30) Priority: **21.04.92 US 871384**

(43) Date of publication of application:
**01.12.93 Bulletin 93/48**

(84) Designated Contracting States:
**AT BE CH DE DK ES FR GB GR IE IT LI LU MC NL PT SE**

(71) Applicant: **MLI ACOUISITION CORP. II**
**781 Chestnut Ridge**
**Morgantown, West Virginia 26505-4310(US)**

(72) Inventor: **Wick, John**
**8 Blue Stem Road**
**Essex Junction, Vermont 05452(US)**

(74) Representative: **Mossmark, Anders et al**
**Albihn West AB**
**Box 142**
**S-401 22 Göteborg (SE)**

(54) **A hydrogel applicator and methods of making same.**

(57) Hydrogel applicators are disclosed including a release liner (2) with periodic indentations (5) filled with hydrogel (1), and a carrier layer (4) for carrying the hydrogel onto the skin, with the carrier layer secured to the hydrogel, so that when the release layer is removed from the carrier layer, the hydrogel is removed from the indentation in the release liner and can be applied to the skin along with the carrier layer. In a preferred embodiment, the carrier layer is a thin film (7), and is in turn carried by a temporary backing sheet (9) which assists in carrying the film and the hydrogel to the skin, and is then removed therefrom.

FIG. 1

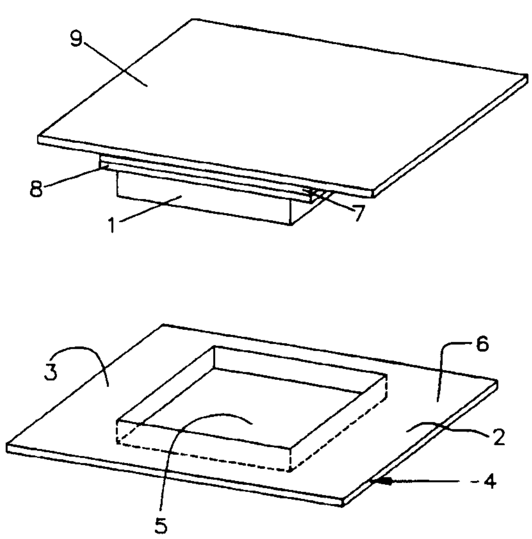


EP 0 571 700 A1

The present invention generally relates to the field of wound dressings. More particularly, the present invention relates to wound dressings for the application of hydrogels. Still more particularly, the present invention relates to processes for the efficient manufacture of such wound dressings.

Hydrogels have become increasingly popular as a means for treating wounds including severe burns because they provide a number of important advantages. Hydrogels provide a barrier to bacteria and other contaminants while at the same time allowing the wound to receive air and moisture. These gels also have a particular utility for use as systems for drug delivery. They may also act to absorb certain secreted waste materials generated during the healing process. Finally, hydrogels also offer a great deal of comfort to the patient, and particularly to the sensitive skin surrounding a wound in that they are soft, pliable, moist and breathable. There are a number of issued U.S. patents which describe such wound dressings employing hydrogels. These include U.S. Patent Nos. 5,002,792; 5,000,955; 4,979,446; 4,948,575; 4,904,247; 4,871,490; 4,767,619; and 4,552,138.

Hydrogels have been prepared, for example, in sheet-like or pad-like forms. These hydrogels are usually at least partially cured, and thus have some rigidity and fixed structure. However, these hydrogel pads are difficult to use. Hydrogel pads are thus often delicate, and tend to break under their own weight much like a tray-full of jello would break if lifted at the edges from its holding pan.

One solution to this problem is discussed in U.S. Patent No. 4,991,574, which discloses the use of a silicone elastomer film which is laminated to a silicon gel on its distal surface. The elastomer helps to provide some structural integrity and continuity to the gel in order to avoid tearing and fragmentation. While this represents a definite improvement, it is still rather difficult to use the hydrogel/elastomer laminate thereof. Moreover, the hydrogels in accordance with the '574 patent are subjected to separate curing and sterilizing steps. As a result, the hydrogel may be overcured.

Thus, there still remains a significant need for improvements in the field of hydrogel applicators and bandages, particularly to improve their ease of construction and use for application to a wound. The present invention provides such advantages.

One of the objects of the present invention is to provide a hydrogel applicator which is economical and convenient to manufacture, as well as easy to use.

Specifically, in accordance with one embodiment of the present invention there is provided a hydrogel applicator comprising a release layer, the release layer including at least one indentation for retaining the hydrogel, the release layer further including an outer surface and an inner surface, and a carrier layer for carrying the hydrogel onto the skin of a patient, the carrier layer being secured to the hydrogel and being releasably secured to the inner surface of the release layer, whereby upon removal of the release layer from the carrier layer the carrier layer can carry the hydrogel for application to the patient.

In accordance with one embodiment of the hydrogel applicator of the present invention, the carrier layer comprises a thin film. In a preferred embodiment, the carrier layer includes an inner surface in contact with the hydrogel and an outer surface, and the applicator includes a backing layer in contact with the outer surface of the carrier layer for temporarily supporting the carrier layer and the hydrogel upon removal of the release layer. Preferably, the backing layer has a greater surface area than the carrier layer, thereby providing an extended peripheral area of the backing layer beyond the outer periphery of the carrier layer.

In a highly preferred embodiment, the applicator also includes adhesion means disposed in the extended peripheral area between the backing layer and the release layer, the adhesion means securing the backing layer to the inner surface of the release layer. Preferably, the adhesion means is a peelable heat seal.

Another object of the present invention is to provide a process for making a convenient hydrogel applicator or bandage. In accordance with this embodiment of the present invention, there is provided a method of manufacturing a hydrogel applicator comprising providing a release layer having an outer surface and an inner surface, the release layer including at least one indentation therein, filling said at least one indentation with the hydrogel in liquid form, providing a carrier layer, and securing the carrier layer to the inner surface of the release layer, so as to enclose the hydrogel between the carrier layer and the release layer. In a particularly preferred embodiment of the method of the present invention, at least one indentation is made by embossing an indentation to a substantially planar release layer, prior to filling the indentation with the hydrogel.

In accordance with a preferred embodiment of the method of the present invention, the method includes simultaneously at least partially curing and sterilizing the enclosed hydrogel.

In accordance with another embodiment of the method of the present invention, the method further includes producing the release layer including at least one indentation by embossing a substantially planar release layer prior to filling the indentation therein with the hydrogel.

Preferred embodiments of the present invention will be described in greater detail with reference to the accompanying drawings, wherein like members bear like reference numerals and wherein;

FIG. 1 is a perspective, exploded view of the separated components of a preferred embodiment of the present invention;

FIG. 2 is a plan side view, in cross section of a preferred embodiment of the device according to the present invention as shown in FIG. 1;

FIG. 3 is a plan side view, in cross-section of another embodiment of the device according to the present invention;

FIG. 4 is a plan side view, in cross-section, of another embodiment of the device according to the present invention;

FIG. 5 is a schematic representation of a preferred embodiment of the method of the present invention; and

FIG. 6 is a top, partial view of a step in the method of the present invention in which the release layer is being prepared.

Referring to FIG. 1 and FIG. 2, the present invention can be used in conjunction with conventional hydrogels 1 such as those formed using the following water soluble or water insoluble gums or resins, with or without known crosslinking agents: agarose, alginates, alkyl and hydroxyalkylcellulose, amylopectin, arabinoglactin, carboxymethylcellulose, carrageenan, eucheuma, fucoidan, furcellaran, gelatin, guar gum, gum agar, gum arabic, gum ghatti, gum karaya, gum tragacanth, hydroxethyl cellulose, hydroxypropyl cellulose, hypnea, keratin laminaran, locusts bean gum, pectin, polyacrylamide, poly(acrylic) acid and homologs, polyethylene glycol, poly(ethylene) oxide, poly(hydroxyalkyl) methacrylate, polyvinyl alcohol, polyvinylpyrrolidone, propylene glycol alginate, starch and modified analogs, tamarind gum, n-vinyl lactam polysaccharides, and xantham gum. In addition, such hydrogels can also be formed by the copolymerization and crosslinking of both hydrophilic and hydrophobic monomers, such as hydroxyalkyl esters of acrylic and methacrylamide, and n-vinyl-2-pyrrolidone, alkyl acrylates and methacrylates, vinyl acetate, acrylonitrile and styrene.

Hydrogels 1 in accordance with the present invention can be prepared as a liquid, paste, semi-solid or solid and then inserted, placed, poured or added to the indentation 5 provided in the release layer 2 which is provided to retain and/or shape the hydrogel 1.

Release layer 2 has an inner surface 3 and an outer surface 4 which may be opposing sides of a single film of, for example, a polymer. These surfaces may also be the exposed sides of two or more discreet layers joined or laminated together to form a single layer. Release layer 2 is basically intended to protect hydrogel 1, as well as any other layers or other material used in the device of this invention, prior to use, and to render them transportable while not interfering with their ultimate application.

In accordance with one aspect of this invention, a hydrogel applicator is provided by which a carrier layer 7, such as a thin film or the like, is applied to the skin along with the hydrogel 1. That is, the carrier layer 7 "carries" the hydrogel 1 onto the skin, and protects it after application thereto. A preferred construction for the hydrogel applicator hereof includes a backing layer 9 which not only temporarily "carries" both the carrier layer 7 and the hydrogel 1, but also, in combination with the release layer 2, provides an overall package for the hydrogel applicator prior to its use. In a preferred such embodiment, the release layer 2 is heat sealed to the backing layer 9 for such purposes.

Release layer 2 can comprise various layers, including paper or paper-containing layers or laminates; various thermoplastics, such as extruded polyolefins, such as polyethylene; various polyester films; foil liners; other such layers, including fabric layers, coated or laminated to various polymers, as well as extruded polyethylene, polyethylene terephthalate, various polyamides, and the like. However, release layer 2 must have an inner surface 3 which is releasable with respect to hydrogel 1 and layers with which it is intended to be initially in contact, such as adhesive layer 8 and/or carrier layer 7. Therefore, when release layer 2 is composed of a material or materials which would not readily release from other layers following adhesive and/or heat sealing, a release coating should be included on inner surface 3 thereof. For example, a release coating is needed when release layer 2 is comprised of, for example, paper. This can be done in a conventional manner, such as by including a silicone or Teflon coating on inner surface 3. However, a particularly preferred embodiment of release layer 2 includes a laminate of an outer foil layer and an inner layer or facing layer of plastic, such as polyethylene or the like. The outer exposed portion of the outer foil layer is the outer surface 4 of release layer 2 and the facing layer of plastic is the inner surface 3 of release layer 2. A separate release coating may not be necessary when release layer 2 is so constructed.

The hydrogel applicator of the present invention includes a carrier layer 7, upon which is maintained hydrogel 1. Carrier layer 7 must ensure that hydrogel 1 will remain associated with the carrier layer 7 upon separation of release layer 2 therefrom.

Carrier layer 7 itself should be flexible enough to generally follow the contour of the area of the host or patient where the device is to be applied. On the other hand, it should have enough strength and substance so as to serve its function of carrying the hydrogel, without wrinkling or breaking. The actual material from which the carrier layer 7 can be produced can therefore include a variety of different materials. Some suitable materials for this layer include, for example, polyethylene, polypropylene, polyvinylidene chloride, polyethylene terephthalate, polyesters, polyamides, and others, as well as laminates of two or more of these layers with each other or with additional layers, such as foil, paper, various fabrics, etc., but in these cases preferably with the polymer layer on the inside, i.e. the facing layer in contact with and thereby carrying the hydrogel 1. Therefore, in the preferred embodiment of the invention, the carrier layer 7 comprises a laminate of an outer foil layer and an inner layer or facing of plastic, such as polyethylene or the like, with low density polyethylene being preferred.

The backing layer 9, which is discussed in more detail below, has as its principal purpose the "carrying" of a carrier layer 7, such as a film layer, which, in turn, will carry the hydrogel onto the patient's skin. The film layer is thus affixed to the inner surface of the backing layer 9 either by being directly extruded onto that surface, or by means of an additional adhesive layer or the like. The carrier layer 7 is preferably a film layer, which is an extremely thin and flimsy layer of material, and which may be either occlusive or non-occlusive in nature. It thus requires the use of a backing layer 9 to handle the film layer, as well as a hydrogel affixed thereto. The film layer or "skin" is not only a flimsy material, but it preferably allows air and moisture vapor to pass therethrough, although it will not permit the passage of bacteria or other undesired elements or materials. It is preferred that carrier layer 7 thus be composed of various thin, plastic materials, or it can comprise an extremely thin foil layer, or layers of other non-woven materials, most particularly comprising a material which is sufficiently thin and flexible to be conformable to the skin, and thus preferably will be thinner than about 2 mils, preferably less than about 1.5 mils, and most preferably 1 mil or less.

These film layers are preferably thermoplastic materials which are at least partially elastomeric in nature. They will therefore exhibit a high degree of elongation (preferably greater than about 130% elongation), and will thus exhibit excellent conformability characteristics without having the tendency to exhibit significant memory characteristics, although they will have some degree of recovery when stretched, for example. In terms of being breathable films it is preferred that these materials, in addition to permitting air to pass therethrough, will also permit moisture vapor to pass through them, at least more readily than is the case with materials such as polyethylene, for example. All of these film layers must be occlusive, in at least one respect, that is with respect to particulates, in order to protect the wound, etc. However, their overall occlusive characteristics can then vary, depending upon the ultimate use intended for them in each particular case. In general, however, it is preferred that films be employed which are permeable to various glycols, such as polyethylene glycols, but rather occlusive films can also be employed in selected circumstances, including, for example, 1 and 2 mil layers of ethylene-vinyl acetate copolymers, or various nylon or polyester films. In addition, laminated or coated films could also be utilized, such as by employing a non-occlusive film such as those discussed above, which is fully or partially selectively coated with an occlusive film.

The various thermoplastic films themselves can generally be produced with either a matte, glossy, or a clear surface, which is obtained by selection or modification of the surface of the chilling roller generally used downstream of the film extruder from which the film is extruded, and they can include various colors, such as skin color, as well as fillers, such as $TiO_2$, clay, or other such materials for the purpose of rendering the film opaque, and various organic additives, odor inhibitors, and/or various medications, etc., directly on the surface thereof.

From the commercial viewpoint, one of the most successful high moisture vapor permeable medical grade elastomeric films has been one of a series of products marketed by Bertek, Inc. under the designation "Medifilm 800." These films are extruded from a class of elastomeric resins which are polyether block amides, commercially designated by the trademark PEBAX. The structure of these polymers can be generally represented by the formula:

$$HO\left(\underset{O}{C}-PA-\underset{O}{C}-PE-\underset{O}{O}\right)NH$$

in which PA represents a relatively rigid polyamide segment and PE represents a relatively soft polyether segment. In this manner the extruded film products have high strengths in terms of high tear and abrasion

resistance, and at the same time provide a high degree of comfort or conformability, as well as moisture vapor permeability. The physical properties of two typical medical grade PEBAX films having a thickness of 1 mil are set forth in Table 1 herein.

TABLE I

| PROPERTIES | FILMS | |
|---|---|---|
| | 810 | 827 |
| Tensile strength - psi (ASTM D882) | 3210 | 2200 |
| % Elongation | 430 | 800 |
| Modulus @ 50% elongation | 1600 | 900 |
| Initial tear resistance - lbs. (ASTM D-1004) | 0.65 | 0.6 |
| MUTR -g/m$^2$/24 hrs. (ASTM E-96) 37.8 C/90% R.H. | 1675 | 2200 |

In addition, other such film layers can comprise thermoplastic polyurethanes which also meet the above requirements. These include such commercial polyurethane compositions as Dow Chemical Company's PELLETHANE, including its 2363-80AE grade thereof; K. J. Quinn's Q-THANE; B. F. Goodrich's ESTANE; Mobay Chemical Company's TXIN; and others. Furthermore, these film layers 10 can also comprise various polyesters, such as the copolymers of various cyclic polyesters including DuPont's HYTREL, including its 4056 grade thereof, and General Electric's LOMOD, both of which are copolymers of polyether prepolymers and polybutylene terephthalate and polyisobutyl terephthalate, respectively, as well as Eastman Chemical's PCCE.

In order to adequately maintain the carrier layer 7 on the surface of the skin of the host, the only additional element required is a layer of adhesive 8 on the underside of carrier layer 7, such as a film layer, as can best be seen in FIGS. 1 and 2.

As noted above, the film layer can be directly extruded onto the surface of the backing layer 9, and will adhere to that surface by means of adhesively melt-forming on that surface. On the other hand, it is possible to maintain the carrier layer 7 on the backing layer 9 by means of an additional layer of adhesive 10 (see FIG. 4) on the underside of the carrier layer 7. The principal requirement is that the adherence of the carrier layer 7 to the backing layer 9 has sufficient strength to maintain the carrier layer on the backing layer when the release layer 2 is separated from the backing layer 9, while at the same time the bond between the carrier layer 7 and the backing layer 9 is sufficiently weak in relative terms, so that it will readily peel off or be removable from the backing layer 9 after the carrier layer 7 with the hydrogel 1 thereon has been applied to the skin by means of the adhesive layer 8 discussed below.

As for the composition of the adhesive layer 10 which is employed, if desired, between the carrier layer 7 and the backing layer 9, this can be a pressure-sensitive adhesive, such as a crosslinkable acrylic copolymer, etc., or it can be a heat-sealable adhesive or the like. Where acrylic copolymers are used, for example, essentially the same acrylic copolymers as are discussed below in connection with the adhesive layer 8 can be applied. However, the adhesion of the adhesive layer 10 itself can be reduced, for example, as compared to that of adhesive layer 8, by adding greater amounts of crosslinking additives thereto, and/or by utilizing coating weights and/or pigments or viscosities therefor.

Adhesive layer 8 serves to assist in the temporary retention of release layer 2 in intimate contact with carrier layer 7 until the applicator of the present invention is used. Adhesive layer 8 may also assist in retaining hydrogel 1 and any associated agents within the confines of the applicator until use. However, adhesive layer 8 has as a principal function retaining the applicator in intimate contact with the skin or wound of a patient when release layer 2 is "peeled away". Another principal function of adhesive layer 8 is retention of hydrogel 1 in intimate contact with carrier layer 7 during use of the applicator, and in retaining hydrogel 1 within the confines of the applicator when in use.

It is not necessary that adhesive layer 8 be composed of a single continuous adhesive component or a single adhesive as illustrated in FIG. 2. For example, a first adhesive may be sized and shaped so that it will exactly fit hydrogel 1. A gap may then be provided, and a second adhesive in the shape of a ring or band 11 may be provided around the periphery. This ring or band may be composed of the same, or a different adhesive and may, in fact, be composed of a mixture of adhesives.

Particularly preferred adhesives useful in accordance with the present invention are those which are hypoallergenic. Adhesives in accordance with the present invention may include esters of acrylic or methacrylic acid and alcohols (e.g. n-butanol, n-pentanol, isopentanol, 2-methyl butanol, 1-methyl butanol,

1-methyl pentanol, 2-methyl pentanol, 3-methyl pentanol, 2-ethyl butanol, isooctanol, n-decanol, or n-dodecanol esters thereof), alone or copolymerized with ethylenically unsaturated monomers such as acrylic acid, methacrylic acid, acrylamide, methacrylamides, N-alkoxymethyl acrylamides, N-alkoxymethyl methacrylamides, N-t-butylacrylamide, itaconic acid, vinyl acetate, N-branched alkyl maleamic acids wherein the alkyl group has 10 to 24 carbon atoms, glycol diacrylates, or mixture of these monomers; natural or synthetic rubbers such as silicon rubber, styrenebutadiene rubber, butylether rubber, neoprene rubber, nitrile rubber, polyisobutylene, polybutadiene, and polyisoprene; polyurethane elastomers, vinyl polymers, such as polyvinyl alcohol, polyvinyl ethers, polyvinylpyrrolidone, and polyvinyl acetate; urea formaldehyde resins; phenol formaldehyde resins; resorcinol formaldehyde resins; cellulose derivatives such as ethyl cellulose, methyl cellulose, nitrocellulose, cellulose acetate butyrate and carboxymethyl cellulose; and natural gums such as guar, acacia, pectina, starch, destria, gelatin, casein, etc. The adhesives may also be compounded with tackifers and stabilizers as is well-known in the art.

To produce the hydrogel applicator of the present invention, a release layer 2 having an indentation 5 therein for retaining hydrogel 1 must be provided. Of course, release layer 2 can be cut or molded such that it includes indentation 5. However, in a preferred embodiment, sheet or roll of material is printed, pressed, and/or embossed so as to provide indentation 5. Embossing may be accomplished by conventional methods which are well-known to those of ordinary skill in this art.

Thereafter, indentation 5 is filled with hydrogel 1. Release layer 2, including indentation 5 filled with hydrogel 1, is then brought into face-to-face contact with carrier layer 7 and, more particularly, with adhesive layer 8 and carrier layer 7, preferably a film layer, interspersed therebetween. In a preferred embodiment, a separate backing layer 9 is employed to carry both the carrier layer 7 (in this case, again preferably a film layer) and the hydrogel 1. The backing layer 9 itself in this instance can thus comprise a material which, while still flexible, is far less flexible and pliable than the film layer, and has sufficient strength and substance to carry both the film layer and the hydrogel 1 without wrinkling or breaking. The actual material of the backing layer 9 can include a variety of different materials. Some suitable materials for this layer include, for example, polyethylene, polypropylene, polyvinylidene chloride, polyethylene terephthalate, polyesters, polyamides, and others, as well as laminates of two or more of these layers with each other or with additional layers, such as foil, paper, various fabrics, etc., but in these cases, preferably with the polymer layer on the inside, i.e., the facing layer in contact with and thereby carrying the film layer. Thus, in a preferred embodiment of the invention, the backing layer 9 comprises a laminate of an outer foil layer and an inner layer or facing of plastic, such as polyethylene or the like with low-density polyethylene being particularly preferred. In this embodiment, the backing layer 9 has a larger surface area than that of the carrier layer 7, and leaves an extended peripheral area around the entire outer periphery thereof. Thus, the backing layer 9 will preferably have an overall surface area substantially the same as that of the release layer 2. When release layer 2 and backing layer 9 are delaminated, the adhesive layer 8 remains on the carrier layer 7, and upon subsequent application to the skin, and removal of the backing layer 9, the adhesive layer 8 will then retain the applicator of the present invention, namely, the carrier layer 7 and the hydrogel 1, in contact with the skin.

In addition, the adhesive layer 8 being in contact with the surface of the release layer 2 surrounding the indentation 5 thereon, thus retaining these layers in intimate contact with each other, heat seals may also be provided between release layer 2 and backing layer 9 at a location external to the carrier layer 7; i.e., within the extended peripheral area discussed above. This heat seal is a so-called "peelable seal," which is represented by reference numeral 14 in FIG. 2, between backing layer 9 and release layer 2. This is often best achieved by using a release layer 2 and a backing layer 9 which are composed of a plurality of layered materials, the innermost of which is suitable for a facing layer. In this regard, it should be noted, for example, that where the two facing layers comprise inner polyethylene layers, the creation of such a "peelable seal" 14 is then accomplished in the manner described in U.S. Pat. No. 4,710,191, the disclosure of which is incorporated here by reference thereto.

In the '191 patent it is disclosed that a "peelable seal" can be created by the incorporation of a release coating on the inner surface of a layer such as release layer 2 so as to render the heat sealed area then created between these two layers, in this case between release layer 2 and backing layer 9, by weakening the thermal bond created therein. It is also disclosed in the '191 patent that one alternative for achieving this result is to employ two different materials as the facing layers for release layer 2 and backing layer 9, such as polyethylene and polypropylene, etc. In such a case no release coating would be required on the inner surface 3 of release layer 2, at least for the purposes of creating a "peelable seal".

In general, the purpose of this "peelable seal" can be to create an overall sealed "package" with the carrier layer 7 and hydrogel 1 therewithin. The peelable seal can also provide other advantages in terms of segregating the hydrogel and preventing migration of any enhancers, solvents, or the like which might be

incorporated therein, which would then adversely effect the transfer rates of the various drugs contained therein. At the same time, these "peelable seals" permit the ready separation of release layer 2 and backing layer 9. For example, if two polyethylene layers were heat sealed together in the manner discussed above without a release coating, such as a silicon layer therebetween, the two layers would essentially melt together, and such separation would not be readily achieved. Thus the nature of these "peelable seals" is an important feature of the present invention.

Referring to FIG. 4, the above-described adhesive layer 10 on the inner surface of backing layer 9 and in contact with the carrier layer 7, can also extend over the entire inner surface of backing layer 9, thus covering the extended peripheral area beyond the carrier layer 7. In this manner, this adhesive layer 10 can be used as adhesion means, instead of a peelable seal, in the manner shown in FIG. 4 to adhere the backing layer 9 to the release layer 2 throughout the extended peripheral outside of the carrier layer 7.

Referring to FIG. 3, a substantially impermeable occlusive layer 12 may also be used in accordance with the present invention. The occlusive layer 12 may be located between adhesive layer 8 and carrier layer 7. Occlusive layer 12 is specifically intended to prevent the seepage of hydrogel 1 through carrier or film layer 7, and may also be used to prevent the seepage of other agents such as pharmaceutically active ingredients, drug components, enhancers, solvents, and the like which may be introduced with or included within hydrogel 1. Occlusive layer 12, in some embodiments, may also be a part of backing layer 9 when backing layer 9 is produced from a complex of layers, including a facing layer and/or an adhesive layer.

The actual material used for the occlusive layer 12 will depend on the properties of the materials in contact therewith. Some suitable materials include, for example, cellophane, cellulose acetate, ethyl cellulose, plasticized vinyl acetate-vinyl chloride copolymers, ethylene-vinyl, acetate copolymer; polyethylene terephthalate, nylon, polyethylene, polypropylene, polyvinylidene chloride (e.g. SARAN), paper, cloth, and aluminum foil. The material which forms this occlusive layer 12 may be flexible or non-flexible. Preferably, a flexible outer surface layer is employed to conform to the shape of the body member to which the device is attached.

Preferably, the material which forms occlusive layer 12 is a film or a composite film. The composite can be a metallized (e.g. aluminized) film or a laminate of two or more films or a combination thereof, such as a laminate of polyethylene terephthalate and polyethylene or a polyethylene/metallized polyethylene terephthalate/polyethylene laminate can be employed. The preferred polymers include polyethylene, polypropylene, polyvinyl chloride, polyethylene terephthalate, and polyvinylidene chloride (SARAN).

As previously, although briefly discussed, hydrogel 1 may also include other ingredients which are to be administered to the body, and more often, to the skin of a patient being treated with the hydrogel applicator of the present invention. These other ingredients may include drug components or pharmaceutically active ingredients which may include, without limitation, vitamins and minerals as well as other ingredients commonly regarded as nutritional supplements and combinations thereof. Other pharmaceutically active ingredients may include analgesics, anti-inflammatories, antibiotics, laxatives, anorexics, antidiarrhetics, antiflatuents, antimigraine agents, antispasmodics, sedatives, antihyperactives, tranquilizers, antihistamine, decongestants, beta-blockers, cough suppressants, antiseptics and combinations thereof, peptide and protein drugs, such as vassopressin, insulin, leuprolide and thyrotropin-releasing hormone.

The amounts of the intended ingredient or ingredients in the composition is selected according to conventional criteria associated with the individual ingredients. An effective amount of each intended ingredient is specifically contemplated. The term "effective amount," as used with reference to an intended ingredient, should be understood as referring to an amount of the intended ingredient sufficient that to perform the normal function of that ingredient. With respect to pharmaceuticals, a pharmaceutically effective amount is contemplated. A pharmaceutically effective amount is the amount or quantity of a drug, mineral or substance which is sufficient to elicit the required or desired therapeutic response.

Other ingredients may also be included to facilitate the diffusion of the pharmaceutically active ingredient such as preservatives, compounds which facilitate skin permeability and the like. These may include enhancers, which are compatible with the hydrogel, alcohols, glycols, polyols, ionic surfactants or the like. Other active ingredients that may be incorporated in the hydrogel are peptide and protein drugs, and/or other drugs which are solubilized or in the form of miceles.

An overall method for the production of a hydrogel applicator in accordance with the present invention will now be set forth with specific reference to FIGS. 5 and 6 hereof.

Referring specifically to FIG. 5, the release layer 2 of this invention is initially prepared from a flat, continuous release layer liner 2a maintained on supply roll 12. The release layer 2a at this point comprises the various layers discussed in the specification, preferably including a release coating on the inner surface thereof. After traveling across idler rollers 13a and 13b, the release layer 2 is then subject to embossment or the like between embossment wheels 14a and 14b. In this manner, the upper surface of the release layer

2 as shown in FIG. 5 is periodically embossed, so as to attain a configuration such as that shown in FIG. 6, with individual indentations 5 spaced periodically along the upper surface of the release layer 2 with its upper surface 3 including the release coating thereon.

After passing further idler rollers 15a and 15b, the indentations 5 are filled with liquid hydrogel 1 by hydrogel applicator 18, which is adapted to fill all of the indentations 5 on the upper surface of the release layer 2 as shown in FIG. 6. As indicated in the specification, conventional hydrogels are set forth, and can be preferably in a liquid or semi-solid form so as to fill these indentations 5 in the appropriate manner. The release layer can then pass over additional idler rollers 16a and 16b.

In a separate operation, a continuous roll of backing layer 9 is removed from roller 20. Again, the backing layer 9 constitutes the various materials discussed in the specification, preferably including polyethylene and/or low-density polyethylene on its inner surface. Preferably, however, the backing layer 9 includes a composite layer with occlusive layer 12, preferably comprising a polyester layer or the like. After passing over idler rollers 21a and 21b, the upper surface of the backing layer 9 then has a carrier layer 7, such as a HYTREL film layer, extruded onto its surface by means of an extruder 22.

After passing further idler rollers 23a and 23b, a layer of adhesive 8 is then applied to the thin film by means of adhesive coating device 24. After passing further idler rollers 25a and 25b, a temporary release liner 27 is applied from roll 28 onto the surface thereof, and pressed into temporary contact by pressure rollers 28a and 28b. This partial applicator device can be separately stored or maintained for later use; i.e., it is "off-line" with respect to a continuous manufacturing process. When it is used, the layer of temporary release liner can then be removed by means of roller 30, and the adhesively exposed surface of the backing layer 9 passes through idler rollers 31a and 31b, and is then die cut by means of die-cut rollers 28a and 28b, with the excess material being removed to roller 34. Die-cut rollers 28a and 28b are thus set to die cut through the adhesive layer 8 and the carrier layer 7, so that excess adhesive and thin film carrier, i.e., outside of the die-cut areas, can then be by means of roller 34. The die-cut carrier strip is then passed over roller 35 and 36, and can then be applied to the face of the release layer 2 as discussed above. These two layers are thus placed in face-to-face contact and pressed together by means of pressure rollers 38a and 38b.

A peelable heat seal can then be applied between the release layer and the backing layer 9 in an area outside of the carrier layer 7 and the adhesive layer 8, by means of heat seal bars 40a and 40b. The heat sealed continuous devices are then passed over idler rolls 41a and 41b, and then over another set of die-cut rollers 42a and 42b, which die cut the individual devices, with the excess material then being removed to roller 44. The die-cut operation takes place outside of the previously created heat seal. After passing further idler rollers 45a and 45b, the individual devices are separated and passed through curing station 50 for final curing of the devices, which can simultaneously accomplish the sterilization of the contents thereof, after which they can be separately handled or packaged in the manner required. This final step in the manufacturing process can also be carried out "off-line," instead of as a step in the continuous manufacturing process hereof.

The principles, preferred embodiments, and modes of operation of the present invention have been described in the foregoing specification. The invention which is intended to be protected herein, however, is not to be construed as limited to the particular embodiments disclosed, since these are to be regarded illustrative rather than restrictive. Variations and changes may be made by others without departing from the spirit and scope of the invention.

## Claims

1. A hydrogel applicator comprising a release layer including an outer surface and an inner surface, and a carrier layer for carrying said hydrogel onto the skin of a patient, said carrier layer being secured to said hydrogel and being releasably secured to said inner surface of said release layer, said hydrogel application characterized by release layer (1, 2) including at least one indentation (5) for retaining said hydrogel, whereby upon removal of said release layer from said carrier layer said carrier layer can carry said hydrogel for application to said patient .

2. The hydrogel applicator according to claim 1 characterized in that said carrier layer comprises a thin film (7).

3. The hydrogel applicator according to claim 1 or 2 characterized in that said carrier layer (7) includes an inner surface in contact with said hydrogel and an outer surface, and including a backing layer (9) in contact with said outer surface of said carrier layer for temporarily supporting said carrier layer and said

hydrogel upon removal of said release layer.

4. The hydrogel applicator according to claim 3 characterized in that said backing layer (9) has a greater surface area than said carrier layer, thereby providing an extended peripheral area (16) of said backing layer beyond the outer periphery of said carrier layer.

5. The hydrogel applicator according to claim 4, further characterized by adhesion means (10, 14) disposed in said extended peripheral area (6) between said backing layer and said release layer, said adhesion means securing said backing layer to said inner surface of said release layer.

6. The hydrogel applicator according to claim 5 characterized in that said adhesion means comprises a peelable heat seal (14).

7. The hydrogel applicator according to claim 1, 2 or 5 further characterized by an adhesive layer (8) disposed on said inner surface of said carrier layer, said adhesive layer securing said carrier layer to said inner surface of said release layer

8. The hydrogel applicator according to claim 4 characterized in that said backing layer (9) is heat sealed (14) to said inner surface of said release layer in said peripheral area surrounding said at least one indentation for retaining said hydrogel.

9. The hydrogel applicator according to any one of the preceding claims characterized in that said hydrogel (1) includes a drug component.

10. The hydrogel applicator according to claim 7 further characterized by an occlusive layer (12) located between said carrier layer and said adhesive layer, thereby preventing release of said drug through said carrier layer.

11. The hydrogel applicator according to any one of the preceding claims characterized in that said hydrogel (1) is selected from the group consisting of polyethylene oxide, polyvinylpyrrolidone, and mixtures thereof.

12. The hydrogel applicator according to claim 3 characterized in that said backing layer (9) includes an inner surface in contact with said carrier layer and an outer surface, and including an occlusive layer (12) in contact with said outer surface of said backing layer.

13. A method of manufacturing a hydrogel applicator characterized by providing a release layer having an outer surface and an inner surface, providing a carrier layer, and securing said carrier layer to said inner surface of said release layer, so as to enclose said hydrogel between said release layer and said carrier layer, said method characterized by providing at least one indentation (5) in said release layer (2) and filling said at least one indentation (5) with said hydrogel (1) in liquid form.

14. The method according to claim 13 further characterized by simultaneously at least partially curing and sterilizing said enclosed hydrogel (1).

15. The method according to claim 14 characterized in that said step of securing said carrier layer (7) to said inner surface of said release layer (2) includes applying an adhesive (8) to said inner surface of said carrier layer.

16. The method according to claim 13, further characterized by providing said at least one indentation in said release layer (2) by embossing a substantially planar release layer (2), prior to filling said at least one indentation (5) with said hydrogel (1).

17. The method according to claim 13 further characterized by providing a backing layer (9), and securing said carrier layer (7) to said backing layer (9).

18. The method according to claim 17 characterized in that said backing layer (9) has a greater surface area than said carrier layer (7), thus providing an extended peripheral area (6) of said backing layer

beyond the outer perimeter of said carrier layer, and characterized in that said securing of said carrier layer (7) to said inner surface of said release layer (2) comprises adhering said backing layer (9) to said release layer (2) in said extended peripheral area (6).

**19.** The method according to claim 13, 14 or 15 characterized in that said carrier layer comprises a thin film.

**20.** The method according to claim 18, 19 or 20 characterized in that said adhering of said backing layer (9) to said inner surface of said release layer (2) comprises heat sealing (14) said backing layer (9) to said release layer (2) in said extended peripheral area (6).

FIG. 1

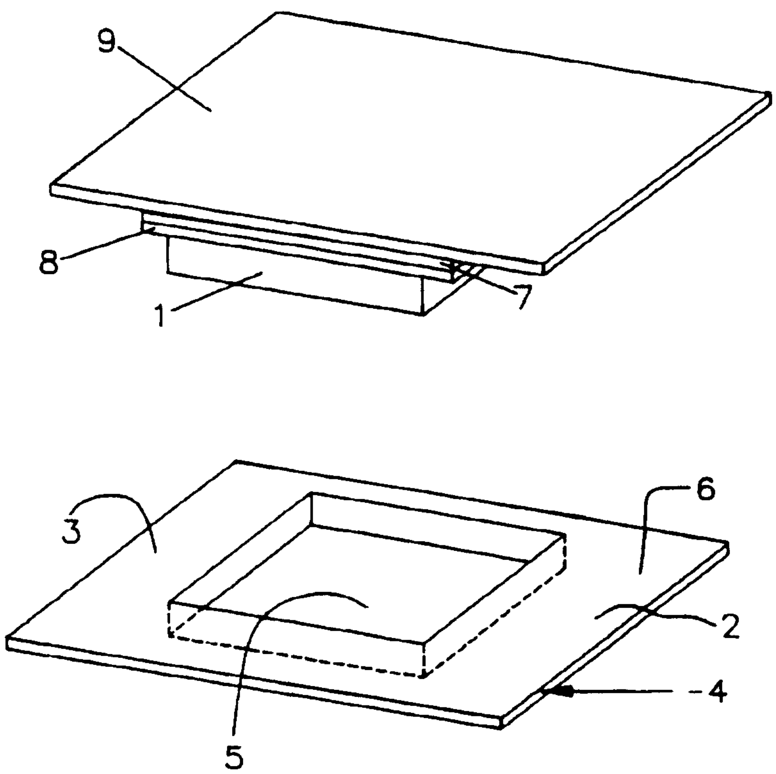

FIG. 2

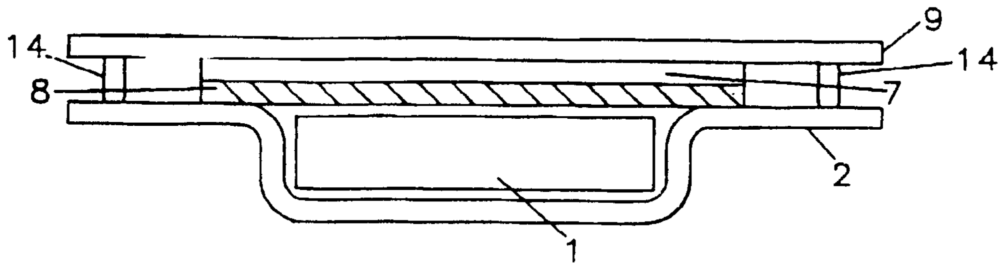


FIG. 3

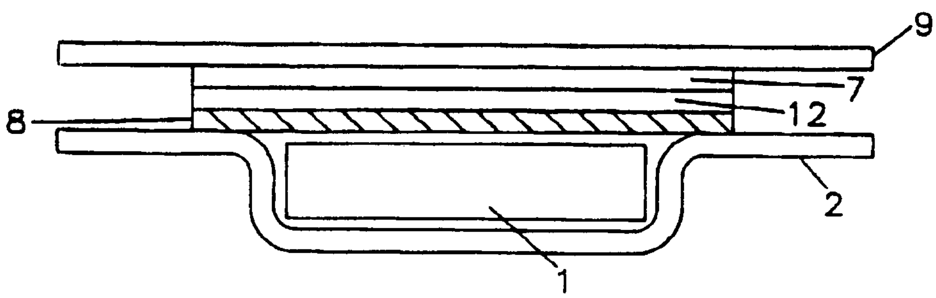


FIG. 4

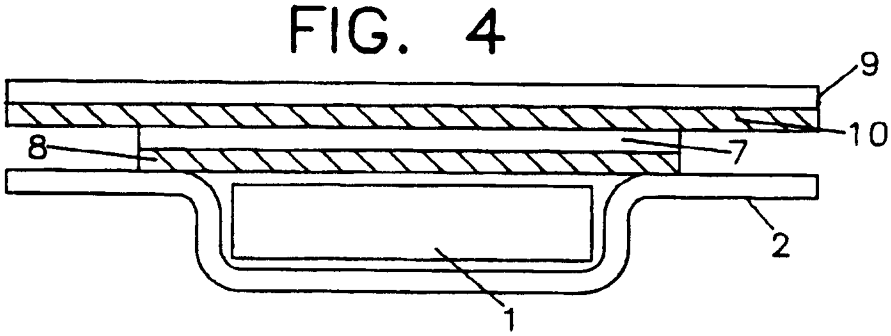

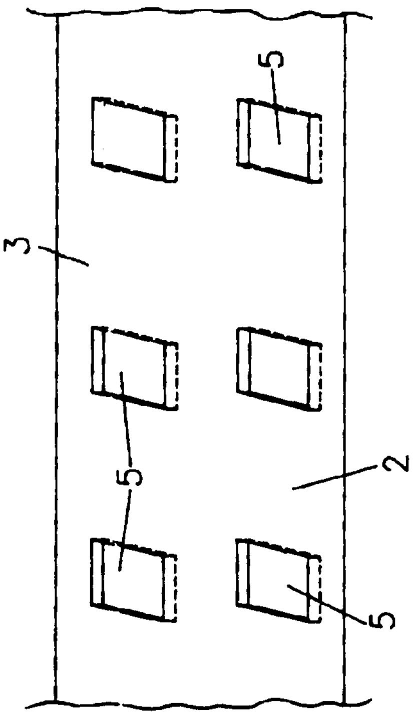
3
5
5
2
5
5

FIG. 6

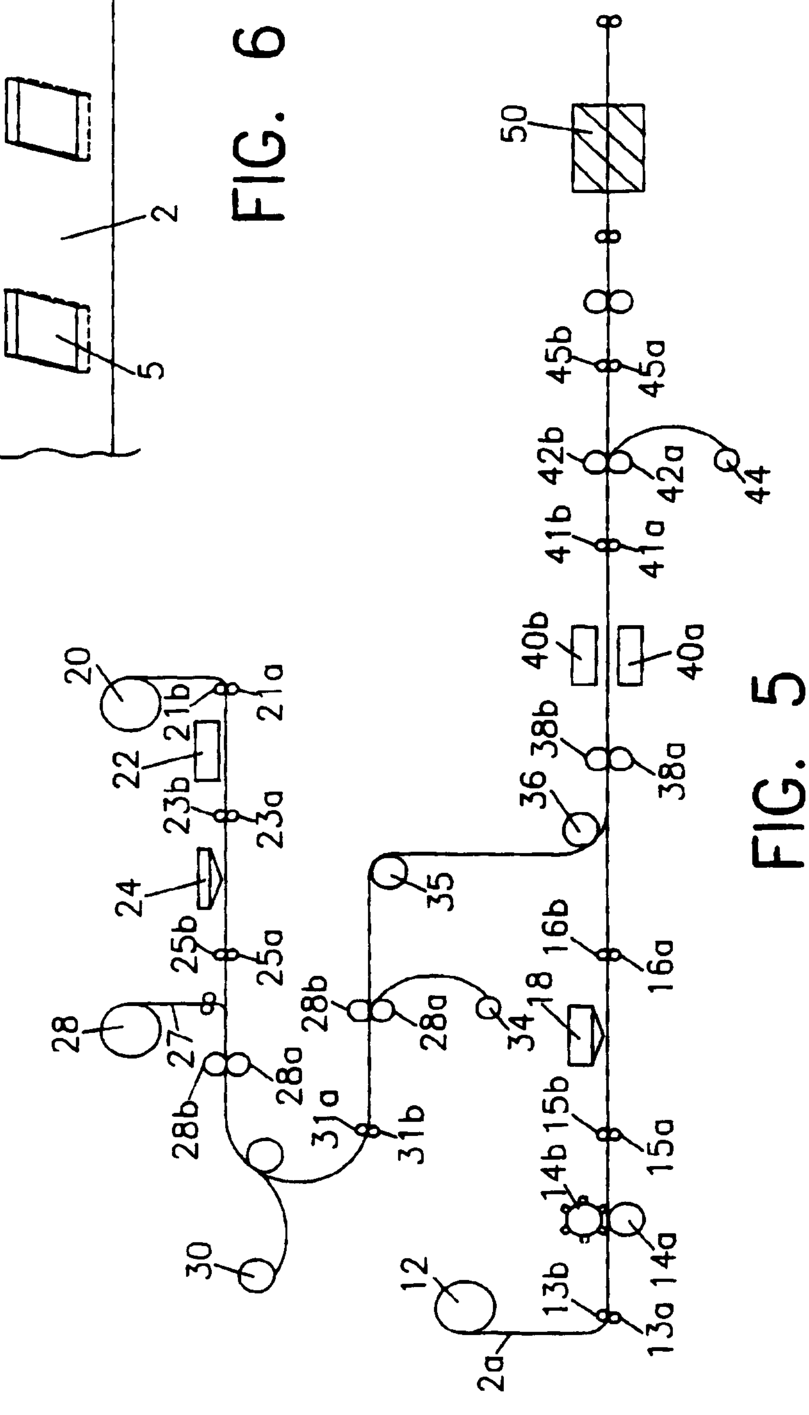
20
21b
21a
22
23b
23a
24
25b
25a
28
27
28b
28a
28b
28a
30
31a
31b
34
35
36
38b
38a
40b
40a
41b
41a
42b
42a
44
45b
45a
50
12
2a
13b
13a
14b
14a
15b
15a
18
16b
16a

FIG. 5

European Patent Office

# EUROPEAN SEARCH REPORT

Application Number

EP 92850235.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP - A1 - 0 455 324 (NDM ACQUISITION CORP.) * Column 3, line 28 - column 9, line 7 * -- | 1-4,7, 9,11, 13,15, 17-19 | A 61 K 9/70<br>A 61 L 15/00 |
| Y | US - A - 4 909 244 (A.J.QUARFOOT et al.) * Column 3, lines 20-28, column 4, lines 44-46, column 5, line 38 - column 7, line 47 * -- | 1-4,7, 9,11, 13,15, 17-19 | |
| A | EP - A1 - 0 040 862 (KEY PHARMACEUTICALS INC.) * Pages 2-4,17-19 * -- | 1-20 | |
| A | EP - A2 - 0 392 845 (MINNESOTA MINING) * Page 4, line 38 - page 5, line 1 * -- | 1-3,9, 13 | TECHNICAL FIELDS SEARCHED (Int. Cl.5) |
| A | EP - A2 - 0 195 643 (MENLEY & JAMES LABORATORIES) * Page 4, lines 15-21, page 7 * ---- | 1,9, 11,13 | A 61 K 9/00<br>A 61 L 15/00 |

The present search report has been drawn up for all claims

| Place of search | Date of completion of the search | Examiner |
|---|---|---|
| VIENNA | 30-07-1993 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document